# DEMANDE DE BREVET EUROPEEN

(11) **EP 0 841 037 A1**
(43) Date de publication de la demande: **13.05.1998**
(21) Numéro de dépôt: 97440103.6
(22) Date de dépôt: 07.11.1997
(51) Int. Cl.: A61B 17/80, A61B 17/17

(54) **Instrumentation de pose d'une lame-agrafe pour ostéotomie de soustraction pour le traitement d'une gonarthrose**

(30) Priorité: 08.11.1996 FR 9613840
(71) Demandeur: PROSEAL, 73000 Chambery (FR)
(72) Inventeur: Legrand, Jean-Jacques, 73000 Chambery (FR)
(74) Mandataire: Arbousse-Bastide, Jean-Claude Philippe

(57) **Abrégé**

Instrumentation de pose d'une lame-agrafe pour ostéotomie de soustraction pour le traitement d'une gonarthrose.

Elle comprend un cadre de positionnement-réduction (2) présentant une ouverture (20) destinée au positionnement d'un bloc de perçage (3) pour la réalisation de trous permettant la pose de la lame-agrafe, l'ouverture (20) est délimitée par deux bords proximal (21) et distal (22), et deux bords latéraux (23) présentant un décrochement (24) similaire à celui de la lame-agrafe, la partie proximale (25) de chacun des bords latéraux (23) comporte des trous superposés (27) destinés au positionnement du cadre de positionnement-réduction (2) au moyen de broches, tandis que le bord distal (26) comporte un trou (29) destiné au passage du mors proximal d'un instrument de serrage permettant de maintenir la compression, tandis que le mors distal est introduit dans un trou pratiqué dans le tibia sous le bord distal du cadre de positionnement-réduction (2) à une certaine distance.

## Description

La présente invention a pour objet une instrumentation de pose d'une lame-agrafe pour ostéotomie de soustraction pour le traitement d'une gonarthrose.

L'ostéotomie de soustraction est une technique opératoire qui consiste à corriger une déformation du genou en varus au niveau du tibia, exceptionnellement en valgus au niveau du fémur.

Cette technique opératoire, pour le traitement d'un genu varum par exemple, consiste à réaliser dans l'épiphyse supérieure du tibia, latéralement du côté externe, une ostéotomie partielle en coin, c'est-à-dire en conservant une charnière du côté interne, puis à réduire le foyer d'ostéotomie par compression, et à maintenir cette correction angulaire jusqu'à l'ostéogenèse.

La difficulté de ce traitement réside dans le maintien en post-opératoire du degré de correction obtenu en per-opératoire.

Pour réaliser le maintien, on utilise actuellement un implant qui peut être une vis-plaque, ou une lame-plaque type col de cygne par exemple, ou encore une lame-agrafe.

Ces différents implants donnent entière satisfaction en ce qui concerne le résultat, toutefois ils présentent les inconvénients de nécessiter un temps opératoire assez long, notamment en ce qui concerne la mise en place de la vis-plaque ou de la lame-plaque, et un abord, ou incision cutanée, important.

La présente invention a pour but de proposer une instrumentation pour la pose d'une lame-agrafe permettant de réduire le temps opératoire et d'en faciliter la pose.

Une lame-agrafe consiste en une agrafe destinée à être positionnée à cheval sur l'ostéotomie après réduction du foyer de celle-ci.

Elle comporte une lame proximale destinée à être impactée dans une fente pratiquée transversalement dans l'épiphyse au-dessus de l'ostéotomie, et deux picots distaux destinés à être impactés dans des trous pratiqués sous l'ostéotomie, la lame et les picots étant réunis par une partie intermédiaire présentant un décrochement afin de créer un décalage dont l'amplitude est fonction de la morphologie du tibia.

L'instrumentation de pose d'une lame-agrafe selon l'invention se caractérise essentiellement en ce qu'elle comprend un cadre de positionnement-réduction présentant une ouverture destinée au positionnement d'un bloc de perçage pour la réalisation des trous permettant la pose de la lame-agrafe, ladite ouverture est délimitée par deux bords proximal et distal, et deux bords latéraux présentant un décrochement similaire à celui de ladite lame-agrafe, c'est-à-dire que les parties proximale et distale dudit cadre sont sensiblement parallèles mais dans des plans différents espacés d'une longueur correspondant à l'amplitude du décalage de la lame-agrafe, ladite partie proximale de chacun desdits bords latéraux comporte des trous superposés destinés au positionnement dudit cadre au moyen de broches, tandis que ledit bord distal comporte un trou destiné au passage du mors proximal d'un instrument de serrage permettant de maintenir la compression, tandis que le mors distal est introduit dans un trou pratiqué dans le tibia sous ledit cadre à une certaine distance du bord distal de celui-ci.

Selon une caractéristique additionnelle de l'instrumentation selon l'invention, le trou de passage du mors de l'instrument de serrage est oblong dans le sens vertical.

Selon une autre caractéristique additionnelle de l'instrumentation selon l'invention, le bord distal du cadre de positionnement-réduction comporte un second trou, situé au-dessus du premier, et destiné à recevoir une broche permettant de maintenir la compression indépendamment de l'instrument de serrage.

Conformément à l'invention, le bloc de perçage comporte à son extrémité proximale une série de trous juxtaposés et percés dans le sens frontal, destinés à permettre le perçage dans l'os de trous pour la préparation à la réalisation de la fente pour loger la lame de la lame-agrafe; et d'autre part à son extrémité distale, deux trous disposés côte à côte, percés dans le sens frontal et d'entraxe correspondant à celui des picots de ladite lame-agrafe, et destinés à loger ceux-ci.

Toujours conformément à l'invention, un gabarit de perçage, destiné à être adapté sur le cadre de positionnement-réduction, comporte un prolongement distal qui déborde le bord distal dudit cadre de positionnement-réduction, et qui est percé de manière frontale d'un trou permettant le perçage dans l'os du trou d'introduction du mors distal de l'instrument de serrage.

Selon un autre mode de réalisation le gabarit de perçage fait partie intégrante du bloc de perçage et consiste en un prolongement distal de celui-ci.

Les avantages et les caractéristiques de la présente invention ressortiront plus clairement de la description qui suit et qui se rapporte au dessin annexé, lequel en représente un mode de réalisation non limitatif.

Dans le dessin annexé :
- la figure 1 représente une vue en perspective d'une lame-agrafe.
- la figure 2 représente une vue en perspective et en éclaté d'une instrumentation de pose d'une lame-agrafe selon l'invention.
- les figures 3a, 3b, 3c, 3d, 3e, et 3f représentent en vues schématiques, différentes phases de la préparation à la pose d'une lame-agrafe au moyen d'une instrumentation selon l'invention.

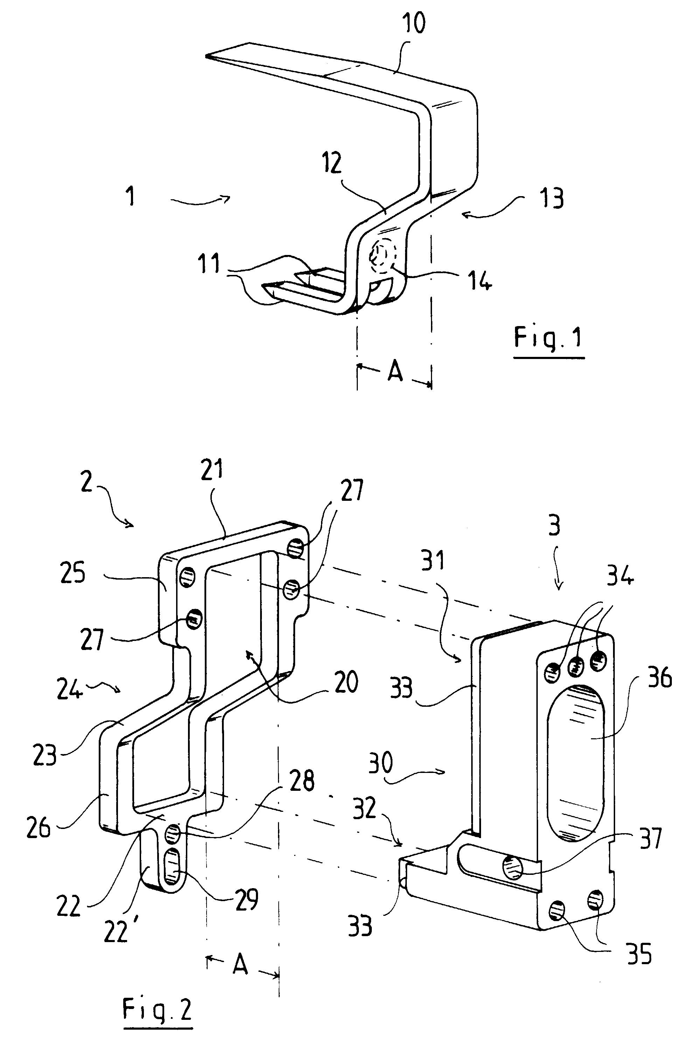

Si on se réfère à la figure 1 on peut voir qu'une lame-agrafe 1 comporte une lame proximale 10 reliée à deux picots distaux 11 par une partie intermédiaire 12 présentant un décrochement 13 d'une certaine amplitude A, qui permet d'épouser approximativement épiphyse et métaphyse supérieures de la face externe d'un tibia, l'amplitude A est variable en fonction de l'anatomie, elle est déterminée en pré-opératoire.

Si on se réfère maintenant à la figure 2 on peut voir une instrumentation de pose d'une lame-agrafe 1 selon l'invention, elle comprend un cadre de positionnement-réduction 2 et un bloc de perçage 3.

Le cadre de positionnement-réduction 2 comporte une ouverture centrale 20 délimitée par un bord proximal 21, un bord distal 22, et deux bords latéraux 23 présentant un décrochement 24 correspondant à l'amplitude A de la lame-agrafe 1, en sorte que leurs parties proximales 25 et distales 26 soient dans des plans parallèles différents.

Les parties proximales 25 des bords latéraux 23 sont percées dans le sens frontal de trous superposés 27, tandis que le bord distal 22 comporte dans sa région médiane un prolongement 22' faisant saillie vers le bas, percé, sensiblement parallèlement aux trous 27, de deux trous superposés 28 et 29, le trou 29 le plus bas étant de section oblongue.

Le bloc de perçage 3 comporte une face 30 d'accouplement au cadre de positionnement-réduction 2 présentant deux parties, une partie distale 32 d'accouplement au bord distal 22 et à la partie distale 26 des bords latéraux 23, et une partie proximale 31 d'accouplement au bord proximal 21 et à la partie proximale 25 des bords latéraux 23, l'adaptation étant réalisée par l'intermédiaire d'un épaulement périphérique 33.

Le bloc de perçage 3 comporte à proximité de son extrémité proximale trois trous frontaux juxtaposés 34, et à proximité de son extrémité distale deux trous frontaux 35, disposés côte à côte et d'entraxe correspondant à celui des picots 11 de la lame-agrafe 1, tandis qu'une lumière 36 est percée centralement entre les trous 34 et 35, permettant un accès visuel dans l'ouverture 20 du cadre de positionnement-réduction 2.

On notera que le bloc de perçage 3 comporte transversalement dans sa partie distale un trou 37 destiné à l'adaptation d'une poignée de maintien, non représentée.

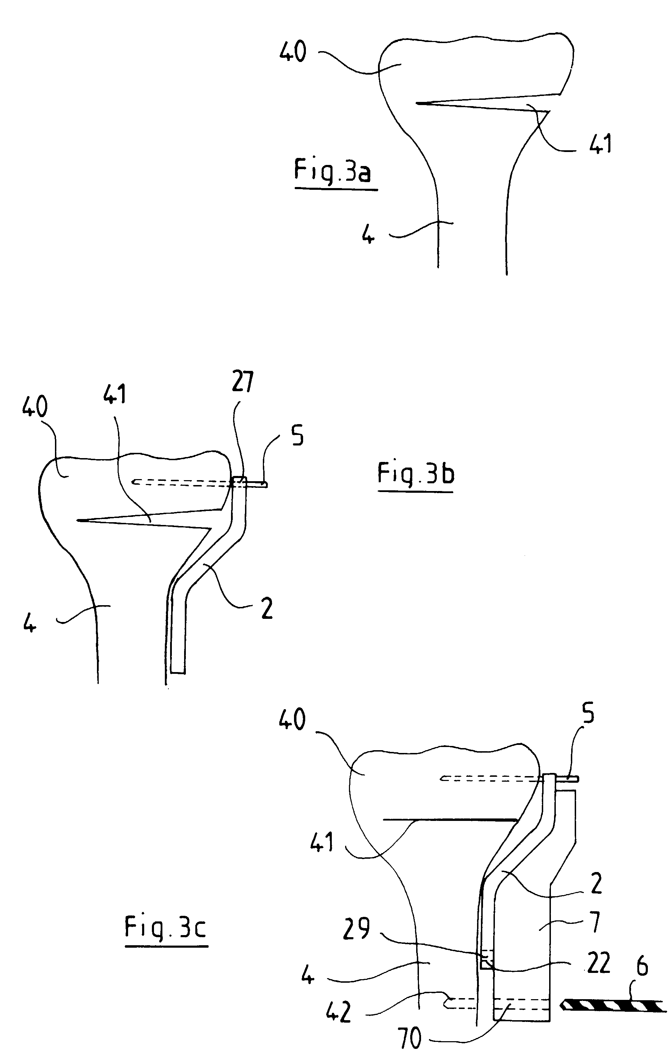

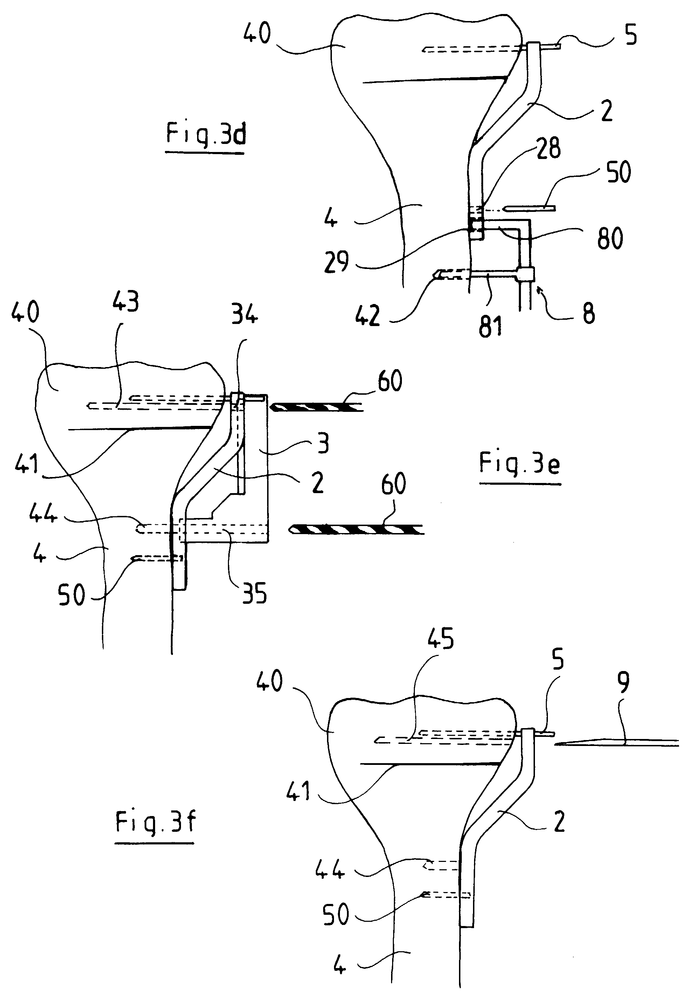

La préparation à la mise en place d'une lame-agrafe 1 au moyen d'une instrumentation selon l'invention va maintenant être décrite à l'appui des figures 3a, 3b, 3c, 3d, 3e et 3f, où est représentée l'épiphyse supérieure 40 d'un tibia 4.

Sur la figure 3a on pratique l'ostéotomie 41 dans l'épiphyse 40, son emplacement est déterminé en y plaçant un cadre de positionnement-réduction 2, non représenté, correspondant à la lame-agrafe 1 à implanter, l'amplitude de celle-ci correspondant à la morphologie du tibia 4.

Sur la figure 3b, le cadre de positionnement-réduction 2 est placé sur l'épiphyse 40 et y est fixé au moyen de deux, voire quatre, broches sans tête 5 introduites dans les trous proximaux 27 du cadre de positionnement-réduction 2.

Sur la figure 3c le foyer de l'ostéotomie 41 est réduit, ce qui est réalisé manuellement, afin de permettre le perçage d'un trou 42 dans le tibia 4 sous le bord distal 22 du cadre 2 au moyen d'une mèche 6 introduite dans le trou distal 70 d'un gabarit 7 assujetti au cadre 2.

On notera que selon une variante, non représentée, le gabarit peut consister en un bloc comportant un trou distal et un crochet proximal destiné à s'accrocher dans le trou distal 29, dont le bord inférieur est avantageusement échancré du côté du tibia afin de permettre un bon accrochage.

Selon une autre variante, non représentée, le bloc de perçage 3 comporte un prolongement distal percé d'un trou permettant le perçage du trou 42.

Sur la figure 3d, la réduction du foyer d'ostéotomie 41 est maintenue, voire accrue, au moyen d'un davier 8 dont le mors proximal 80 est introduit dans le trou 29 du cadre 2 tandis que le mors distal 81 est introduit dans le trou 42 percé dans le tibia 4. Après réduction complète du foyer de l'ostéotomie 41, une broche 50 peut être introduite dans le trou 28 du cadre 2 afin de fixer ce dernier.

D'autre part, il est également possible de percer le trou 42 avant la réduction du foyer d'ostéotomie, qui peut être réalisée au moyen uniquement du davier 8.

Le davier 8 doit donc être de conception robuste, ce qui explique la forme oblongue du trou 29 du cadre 2, afin de permettre le passage du mors proximal 80 qui est de section importante.

Sur la figure 3e, le bloc de perçage 3 est adapté sur le cadre de positionnement-réduction 2, et trois trous proximaux 43, dont un seul est visible sur la figure, et deux trous distaux 44, dont un seul est visible sur la figure, sont percés dans l'épiphyse 40 au moyen d'une mèche 60 introduite successivement dans les trous 34 et 35 du bloc de perçage 3.

On notera que ces opérations de perçage peuvent être réalisées alors que le cadre 2 est maintenu par le davier 8, non représenté.

Enfin, sur la figure 3f, le bloc de perçage 3 est enlevé, et une fente 45 est réalisée au moyen d'un ciseau conformateur 9 qui coupe la matière osseuse séparant les trous proximaux 43, le ciseau conformateur 9 étant de dimensions correspondant à celles de la lame 10 de la lame-agrafe 1.

Il reste alors à impacter la lame-agrafe 1, dont la lame 10 est introduite dans la fente 45, tandis que les picots 11 sont introduits dans les trous distaux 44.

Lorsque la lame-agrafe 1 est en place, le cadre de positionnement-réduction 2 et ses moyens de fixation peuvent être enlevés.

Le maintien de la lame-agrafe 1 peut être accru au moyen d'une vis introduite dans un trou 14, représenté en traits discontinus sur la figure 1, pratiqué dans la partie intermédiaire 12, juste au-dessus des picots 11.

Il va de soi que la présente invention ne saurait être limitée à la description qui précède d'un de ses modes de réalisation, susceptible de subir un certain nombre de modifications sans pour autant sortir du cadre de l'invention.

L'instrumentation objet de la présente invention pourra notamment être utilisée pour la pose d'autres implants utilisés pour les ostéotomies de soustraction, en ne nécessitant que de substantielles modifications.

Dans le cas d'une lame-plaque par exemple, qui diffère d'une lame-agrafe essentiellement en ce que la fixation distale est réalisée au moyen d'au moins une vis au lieu des picots, le bloc de perçage comporte des trous permettant de pratiquer dans l'os les trous destinés à recevoir la ou les vis.

## Revendications

1. Instrumentation de pose d'une lame-agrafe pour ostéotomie de soustraction pour le traitement d'une gonarthrose, ladite lame-agrafe (1) comportant une lame proximale (10) destinée à être impactée dans une fente (45) pratiquée transversalement dans l'épiphyse (40) au-dessus de l'ostéotomie (41), et deux picots distaux (11) destinés à être impactés dans des trous distaux (44) pratiqués sous l'ostéotomie (41), la lame (10) et les picots (11) étant réunis par une partie intermédiaire (12) présentant un décrochement (13) afin de créer un décalage dont l'amplitude (A) est fonction de la morphologie du tibia (4), ladite instrumentation est caractérisée en ce qu'elle comprend un cadre de positionnement-réduction (2) présentant une ouverture (20) destinée au positionnement d'un bloc de perçage (3) pour la réalisation de trous (43, 44) permettant la pose de la lame-agrafe (1), ladite ouverture (20) est délimitée par deux bords proximal (21) et distal (22), et deux bords latéraux (23) présentant un décrochement (24) similaire à celui de ladite lame-agrafe (1), c'est-à-dire que les parties proximale (25) et distale (26) dudit cadre de positionnement-réduction (2) sont sensiblement parallèles mais dans des plans différents espacés d'une longueur correspondant à l'amplitude (A) du décalage de la lame-agrafe (1), ladite partie proximale (25) de chacun desdits bords latéraux (23) comporte des trous superposés (27) destinés au positionnement dudit cadre de positionnement-réduction (2) au moyen de broches (5), tandis que ledit bord distal (26) comporte un trou (29) destiné au passage du mors proximal (80) d'un instrument de serrage (8) permettant de maintenir la compression, tandis que le mors distal (81) est introduit dans un trou (42) pratiqué dans le tibia (4) sous ledit cadre de positionnement-réduction (2) à une certaine distance du bord distal (22) de celui-ci.

2. Instrumentation selon la revendication 1 caractérisée en ce que le trou (29) de passage du mors proximal (80) de l'instrument de serrage (8) est oblong dans le sens vertical.

3. Instrumentation selon la revendication 1 ou la revendication 2 caractérisée en ce que le bord distal (22) du cadre de positionnement-réduction (2) comporte un second trou (28), situé au-dessus du premier (29), et destiné à recevoir une broche (50) permettant de maintenir la compression indépendamment de l'instrument de serrage (8).

4. Instrumentation selon la revendication 3 caractérisée en ce que les trous (28, 29) sont pratiqués dans un prolongement (22') faisant saillie vers le bas du bord distal (22) du cadre de positionnement-réduction (2).

5. Instrumentation selon l'une quelconque des revendications précédentes caractérisée en ce que le bloc de perçage (3) comporte d'une part à son extrémité proximale, une série de trous (34) juxtaposés et percés dans le sens frontal, destinés à permettre le perçage dans l'os de trous (43) pour la préparation à la réalisation de la fente (45) pour loger la lame (10) de la lame-agrafe (1); et d'autre part à son extrémité distale, deux trous (35) disposés côte à côte, percés dans le sens frontal et d'entraxe correspondant à celui des picots (11) de ladite lame-agrafe (1).

6. Instrumentation selon la revendication 5 caractérisée en ce que le bloc de perçage (3) comporte dans sa partie distale un trou transversal (37) permettant l'adaptation d'une poignée de maintien.

7. Instrumentation selon l'une quelconque des revendications précédentes caractérisée en ce qu'un gabarit de perçage (7) est adaptable sur le cadre de positionnement-réduction (2), il comporte un prolongement distal qui déborde le bord distal (22) dudit cadre de positionnement-réduction (2), et qui est percé de manière frontale d'un trou (70) permettant le perçage dans l'os du trou (42) d'introduction du mors distal (81) de l'instrument de serrage (8).

8. Instrumentation selon la revendication 7 caractérisée en ce que le gabarit de perçage comporte un crochet proximal destiné à être introduit dans le trou distal (29) du cadre de positionnement-réduction (2) et destiné au passage du mors proximal (80) de l'instrument de serrage (8), le bord inférieur dudit trou (29) étant échancré du côté du tibia (4).

9. Instrumentation selon l'une quelconque des revendication de 1 à 6, caractérisée en ce que le bloc de perçage (3) comporte un prolongement distal percé dans le sens frontal d'un trou destiné à permettre le perçage dans l'os (4) du trou (42) d'introduction du mors distal (81) de l'instrument de serrage (8).

10. Instrumentation de pose d'une lame-plaque pour ostéotomie de soustraction pour le traitement d'une gonarthrose, ladite lame-plaque comportant une lame proximale destinée à être impactée dans une fente pratiquée transversalement dans l'épiphyse au-dessus de l'ostéotomie, et au moins un trou pratiquée dans sa partie distale, destiné à permettre la fixation de ladite lame-plaque au moyen d'une vis, ladite lame et ladite partie distale étant réunies par une partie intermédiaire présentant un décrochement afin de créer un décalage dont l'amplitude est fonction de la morphologie du tibia, ladite instrumentation est caractérisée en ce qu'elle comprend un cadre de positionnement-réduction présentant une ouverture destinée au positionnement d'un bloc de perçage pour la réalisation de trous permettant la pose de la lame-plaque, ladite ouverture est délimitée par deux bords proximal et distal et deux bords latéraux présentant un décrochement similaire à celui de ladite lame-plaque, c'est-à-dire que les parties proximale et distale dudit cadre de positionnement-réduction sont sensiblement parallèles mais dans des plans différents espacés d'une longueur correspondant à l'amplitude du décalage de la lame-plaque, ladite partie proximale de chacun desdits bords latéraux comporte des trous superposés destinés au positionnement dudit cadre de positionnement-réduction au moyen de broches, tandis que ledit bord distal comporte un trou destiné au passage du mors proximal d'un instrument de serrage permettant de maintenir la compression, tandis que le mors distal est introduit dans un trou pratiqué dans le tibia sous ledit cadre de positionnement-réduction à une certaine distance du bord distal de celui-ci.
